# EUROPEAN PATENT SPECIFICATION

(11) **EP 4 313 238 B1**
(45) Date of publication and mention of the grant of the patent: **12.08.2026**
(21) Application number: 22776453.7
(22) Date of filing: 22.03.2022
(51) Int. Cl.: A61M 25/01, A61M 25/08, A61M 25/09, A61B 5/026, A61B 5/0215, A61M 25/00, A61B 5/145, A61B 5/00, A61B 5/06, A61B 5/01

(54) **VASCULAR ACCESS DEVICE TO REDUCE BUCKLING OF AN INSTRUMENT**
GEFÄSSZUGANGSVORRICHTUNG ZUR REDUZIERUNG DES KNICKENS EINES INSTRUMENTS
DISPOSITIF D'ACCÈS VASCULAIRE POUR RÉDUIRE LE GAUCHISSEMENT D'UN INSTRUMENT

(30) Priority: 23.03.2021 US 202163164976 P
(43) Date of publication of application: 07.02.2024
(73) Proprietor: Becton, Dickinson and Company, Franklin Lakes, NJ 07417 (US)
(72) Inventor: HARDING, Weston, F., Lehi, Utah 84043 (US); SCHERICH, Megan, Salt Lake City, Utah 84115 (US); BURKHOLZ, Jonathan, Karl, Salt Lake City, Utah 84108 (US); LACKEY, John, Valley City, Utah 84119 (US); NEUMANN, Erica, E., Salt Lake City, Utah 84106 (US); HESLOP, Vaughn, Saratoga Springs, Utah 84045 (US); BLANCHARD, Curtis, H., Riverton, Utah 84096 (US); MA, Yiping, Layton, Utah 84040 (US)
(74) Representative: dompatent
(86) International application number: PCT/US2022/021319
(87) International publication number: WO 2022/204123

(56) References cited:
- AU-A1- 2016 265 702
- US-A1- 2018 133 437
- US-A1- 2019 275 302
- US-A1- 2019 275 302
- US-A1- 2020 016 374
- US-A1- 2020 246 590
- US-A1- 2020 246 590
- US-A1- 2021 290 264
- US-B2- 10 238 840

## Description

### CROSS-REFERENCE TO RELATED APPLICATION

The present application claims priority to United States Provisional Application Serial No. 63/164,976, entitled "Vascular Access Device to Reduce Buckling of an Instrument", filed March 23, 2021.

### BACKGROUND

Catheters are commonly used for a variety of infusion therapies. For example, catheters may be used for infusing fluids, such as normal saline solution, various medicaments, and total parenteral nutrition, into a patient. Catheters may also be used for withdrawing blood from the patient.

A common type of catheter device includes a catheter that is over-the-needle. As its name implies, the catheter that is over-the-needle may be mounted over an introducer needle having a sharp distal tip. A catheter assembly may include a catheter adapter, the catheter extending distally from the catheter adapter, and the introducer needle extending through the catheter. The catheter and the introducer needle may be assembled so that the distal tip of the introducer needle extends beyond the distal tip of the catheter with the bevel of the needle facing up away from skin of the patient. The catheter and introducer needle are generally inserted at a shallow angle through the skin into vasculature of the patient.

In order to verify proper placement of the introducer needle and/or the catheter in the blood vessel, a clinician generally confirms that there is "flashback" of blood in a flashback chamber of the catheter assembly. Once placement of the needle has been confirmed, the clinician may temporarily occlude flow in the vasculature and remove the needle, leaving the catheter in place for future blood withdrawal or fluid infusion.

Infusion and blood withdrawal using the catheter may be difficult for several reasons, particularly when an indwelling time of the catheter increase. A fibrin sheath or thrombus may form on an internal surface of the catheter assembly, an external surface of the catheter assembly, or within the vasculature near the distal tip of the catheter. The fibrin sheath or thrombus may block or narrow a fluid pathway through the catheter, which may impair infusion and/or collection of a high-quality blood sample.

The subject matter claimed herein is not limited to embodiments that solve any disadvantages or that operate only in environments such as those described above. Rather, this background is only provided to illustrate one example technology area where some implementations described herein may be practiced.

US 2019/275302 and US 2020/246590 are both directed to fluid transfer devices. The fluid transfer devices both include an introducer (200/910), a catheter (260/960), and an actuator (270/970). The introducer (200/910) defines an inner volume to receive portions of the actuator (270/970) and the catheter (260/960) therein. The actuator (270/970) is movably coupled to the introducer (200/910), with a first portion of the actuator (270/970) disposed outside of the introducer (200/910) and a second portion of the actuator (270/970) disposed within the inner volume and fixedly coupled to a distal end portion of the catheter (260/960). A user can engage the first portion of the actuator (270/970) and move the catheter (260/960) between a first position and a second position, where at least the distal end portion of the catheter (260/960) is disposed within the inner volume when the catheter (260/960) is in the first position and the distal end of the catheter (260/960) is positioned outside the inner volume when the catheter (260/960) is in the second position.

AU 2016/265702 is directed to an insertion tool (10) for inserting a catheter (42) into a patient's body. The insertion tool includes a housing (12), a hollow needle (16), a guidewire (22), and a guidewire advancement assembly (20). The guidewire advancement assembly (20) includes a guidewire lever (24) and guidewire advancement slide (28). Two tabs (24A) of the guidewire lever (24) attach to the slide (28) so that movement of the slide (28) by a user results in corresponding movement of the lever (24). The tabs (24A) also help secure the slide (28) to the housing (12).

### SUMMARY OF THE INVENTION

A vascular access device according to the invention is defined by the features of claims 1 and 15, respectively. Preferred embodiments are defined by the features of the dependent claims. The present disclosure relates generally to vascular access devices and related systems and methods (no methods are claimed). In some embodiments, a vascular access device includes a housing, which includes a proximal end, a distal end, and a slot. In some embodiments, an inner surface of the housing includes a groove disposed within the housing between the proximal end of the housing and the distal end of the housing. In some embodiments, the vascular access device includes an advancement element extending through the slot and configured to move linearly along the slot between a retracted position and an advanced position.

In some embodiments, the vascular access device includes an instrument disposed within the groove. In some embodiments, the instrument includes a first end and a second end. In some embodiments, in response to movement of the advancement element from the retracted position to the advanced position, the second end of the instrument may be advanced beyond the distal end of the housing.

In some embodiments, the vascular access device includes a support feature disposed on top of the groove or within the groove. In some embodiments, the support feature is configured to move distally in response to movement of the advancement element from the retracted position to the advanced position. In some embodiments, the support feature limits displacement of the instrument from the groove.

In some embodiments, the groove may be linear. In some embodiments, the support feature may be disposed on top of the groove. In some embodiments, in response to movement of the advancement element from the retracted position to the advanced position, the advancement element may contact a proximal end of the support feature and push the support feature distally along the groove.

In some embodiments, the vascular access device may include an arm extending from the proximal end of the support feature. In some embodiments, the arm may extend through the advancement element. In some embodiments, in response to movement of the advancement element from the retracted position to the advanced position, the advancement element may slide distally along the arm until the advancement element contacts the proximal end of the support feature.

In some embodiments, in response to movement of the advancement element from the advanced position to the retracted position, the advancement element may pull the arm and the support feature proximally. In some embodiments, a proximal end of the arm may include a hook. In some embodiments, in response in response to movement of the advancement element from the advanced position to the retracted position, the advancement element may catch on the hook and pull the arm and the support feature proximally.

In some embodiments, the support feature may include a spring. In some embodiments, in response to movement of the advancement element from the retracted position to the advanced position, the spring may compress in a distal direction. In some embodiments, the spring may be disposed within the groove and the instrument may extend through the spring. In some embodiments, the spring may be disposed on top of the groove.

In some embodiments, the support feature may include one or more elastomeric discs disposed within the groove. In some embodiments, in response to movement of the advancement element from the retracted position to the advanced position, each of the elastomeric discs may slide distally within the groove. In some embodiments, the support feature may include a telescoping member. In some embodiments, in response to movement of the advancement element from the retracted position to the advanced position, the telescoping member may collapse in the distal direction.

In some embodiments, the support feature may include a strip. In some embodiments,
the strip may include a first end and a second end. In some embodiments, the first end may be coupled to the advancement element and the second end may be configured to roll up in response to movement of the advancement element from the retracted position to the advancement position. In some embodiments, the first end of the strip may include a proximal end of the strip, and the second end of the strip may include a distal end of the strip.

In some embodiments, the support feature may include a cover extending over the groove. In some embodiments, a first side of the cover and/or a second side of the cover opposite the first side of the cover may be coupled to the housing. In some embodiments, the cover may include a slit down a middle of the cover. In other embodiments, the advancement element may include a blade that cuts through the cover in response to movement of the advancement element from the retracted position to the advanced position. In some embodiments, the support feature may include one or more tabs extending over the groove and configured to swivel or bend in response to movement of the advancement element from the retracted position to the advanced position and contact by the advancement element.

In some embodiments, the advancement element may include an arc-shaped channel. In some embodiments, the instrument may extend through the arc-shaped channel. In some embodiments, the first end of the instrument may be fixed. In some embodiments, in response to movement of the advancement element a first distance, the second end of the instrument may be configured to advance distally a second distance. In some embodiments, the second distance may be at least twice the first distance. In some embodiments, the groove may be a first groove. In some embodiments, the inner surface of the housing may include a second groove between the proximal end of the housing and the distal end of the housing and generally parallel to the first groove. In some embodiments, the instrument may extend through the second groove.

In some embodiments, the support feature may be disposed between the first groove and the second groove and may limit displacement of the instrument from the first groove and/or the second groove. In some embodiments, the support feature may be disposed within the first groove and/or the second groove. In some embodiments, the vascular access device may include another support feature disposed within the second groove. In some embodiments, the instrument may extend through the support feature and the other support feature.

In some embodiments, the support feature and/or the other support feature may include the spring. In some embodiments, the support feature and/or the other support feature may include an accordion shape, and in response to movement of the advancement element from the retracted position to the advanced position, the accordion shape may compress in the distal direction.

In some embodiments, the vascular access device includes a tube disposed within the groove. In some embodiments, the instrument extends through the tube. In some embodiments, the tube may include a slit and in response to movement of the advancement element from the retracted position to the advanced position, the advancement element may move distally through the slit. In some embodiments, the advancement element may include the arc-shaped channel, and in response to movement of the advancement element from the retracted position to the advanced position, the tube may slide into the arc-shaped portion.

It is to be understood that both the foregoing general description and the following detailed description are examples and explanatory and are not restrictive of the invention, as claimed. It should be understood that the various embodiments are not limited to the arrangements and instrumentality illustrated in the drawings. It should also be understood that the embodiments may be combined, or that other embodiments may be utilized and that structural changes, unless so claimed, may be made without departing from the scope of the various embodiments of the present invention. The following detailed description is, therefore, not to be taken in a limiting sense.

### BRIEF DESCRIPTION OF THE DRAWINGS

Example embodiments will be described and explained with additional specificity and detail through the use of the accompanying drawings in which:
Figure 1A is an upper perspective view of an example vascular access device, according to some embodiments;
Figure 1B is a longitudinal cross-sectional view of the vascular access device of Figure 1A;
Figure 1C is a cross-sectional view of the vascular access device of Figure 1A along the line 1C-1C of Figure 1A, according to some embodiments;
Figure 1D is an enlarged view of a portion of Figure 1C, according to some embodiments;
Figure 1E is a cross-sectional view of the vascular access device of Figure 1A along the line 1E-1E of Figure 1A, according to some embodiments;
Figure 2A is an upper perspective view of another example vascular access device, according to some embodiments;
Figure 2B is a longitudinal cross-sectional view of the vascular access device of Figure 2A, illustrating an example advancement element in an example retracted position, according to some embodiments;
Figure 3A is a longitudinal cross-sectional view of the vascular access device of Figure 1A, illustrating the advancement element in the retracted position and an example support feature, according to some embodiments;
Figure 3B is a longitudinal cross-sectional view of the vascular access device of Figure 1A, illustrating the support feature pushed by the advancement element in response to the advancement element moving distally towards an example advanced position, according to some embodiments;
Figure 3C is a longitudinal cross-sectional view of the vascular access device of Figure 1A, illustrating the advancement element in the advanced position, according to some embodiments;
Figure 3D is a longitudinal cross-sectional view of the vascular access device of Figure 1A, illustrating the advancement element being retracted proximally from the advanced position towards the retracted position, according to some embodiments;
Figure 3E is a longitudinal cross-sectional view of the vascular access device of Figure 1A, illustrating the advancement element returned to the retracted position, according to some embodiments;
Figure 4A is a longitudinal cross-sectional view of the vascular access device of Figure 1A, illustrating the advancement element in the retracted position and an example spring, according to some embodiments;
Figure 4B is a cross-sectional view of the vascular access device of Figure 1A along the line 1E-1E, according to some embodiments;
Figure 4C is a longitudinal cross-sectional view of the vascular access device of Figure 1A, illustrating the advancement element in the retracted position and the spring, according to some embodiments;
Figure 4D is a longitudinal cross-sectional view of the vascular access device of Figure 1A, illustrating the advancement element in the retracted position and multiple of the springs, according to some embodiments;
Figure 4E is a longitudinal cross-sectional view of the vascular access device of Figure 1A, illustrating the spring within an example groove, according to some embodiments;
Figure 4F is an upper perspective view of an example catheter system, illustrating another example vascular access device that includes the spring, according to some embodiments;
Figure 5 is a longitudinal cross-sectional view of the vascular access device of Figure 1A, illustrating an example accordion shape, according to some embodiments;
Figure 6A is a longitudinal cross-sectional view of the vascular access device of Figure 1A, illustrating the advancement element in the retracted position and an example elastomeric discs, according to some embodiments;
Figure 6B is an enlarged upper perspective view of an example one of the elastomeric discs, illustrating an example instrument extending therethrough, according to some embodiments;
Figure 6C is a longitudinal cross-sectional view of the vascular access device of Figure 1A, illustrating the advancement element in the retracted position and the elastomeric discs, according to some embodiments;
Figure 6D is an enlarged upper perspective view of an example one of the elastomeric discs, illustrating the instrument extending therethrough, according to some embodiments;
Figure 7 is a longitudinal cross-sectional view of the vascular access device of Figure 1A, illustrating the advancement element in the retracted position and example telescoping members, according to some embodiments;
Figure 8 is a longitudinal cross-sectional view of the vascular access device of Figure 1A, illustrating the advancement element in the retracted position and example strips, according to some embodiments;
Figure 9A is a cross-sectional view of the vascular access device of Figure 1A along the line 1C-1C of Figure 1A, illustrating example covers, according to some embodiments;
Figure 9B is an enlarged view of a portion of Figure 9A, according to some embodiments;
Figure 9C is a longitudinal cross-sectional view of the vascular access device of Figure 1A, illustrating the covers, according to some embodiments;
Figure 9D is a longitudinal cross-sectional view of the vascular access device of Figure 1A, illustrating example elongated covers, according to some embodiments;
Figure 9E is a cross-sectional view of the vascular access device of Figure 1A along the line 1C-1C, illustrating the elongated covers, according to some embodiments;
Figure 10 is a longitudinal cross-sectional view of the vascular access device of Figure 1A, illustrating the advancement element in the retracted position and example tube, according to some embodiments;
Figure 11A is a cross-sectional view of the vascular access device of Figure 1A along the line 1C-1C, illustrating example tabs, according to some embodiments;
Figure 11B is a longitudinal cross-sectional view of the vascular access device of Figure 1A, illustrating the advancement element in the retracted position and the tabs, according to some embodiments; and
Figure 12 is a longitudinal cross-sectional view of the vascular access device of Figure 1A, illustrating the advancement element in the retracted position and the tube, according to some embodiments.

### DETAILED DESCRIPTION

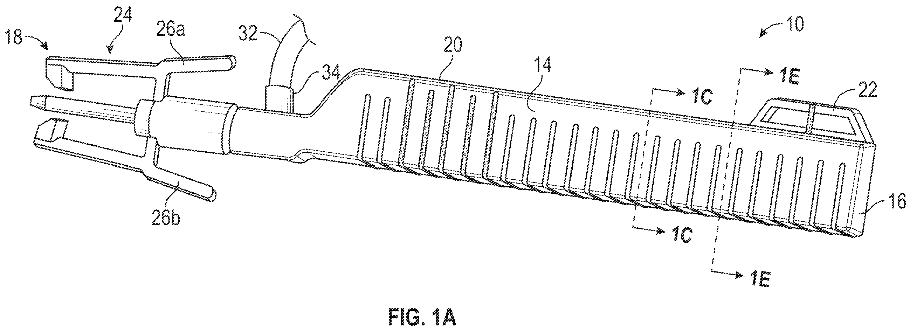

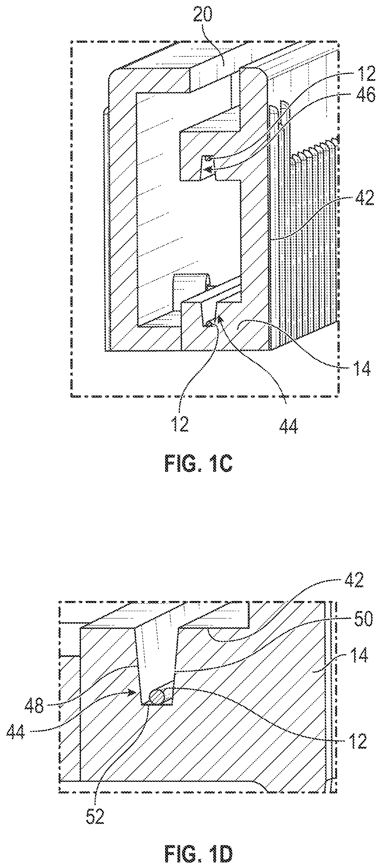

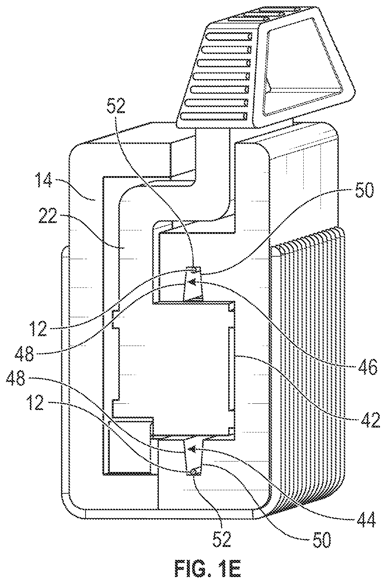

Referring now to Figures 1A-1D, in some embodiments, a vascular access device 10 may be configured to deliver an instrument 12 through a catheter of a catheter assembly. In some embodiments, the instrument 12 may be advanced through the catheter to push past any occlusions in the catheter or vasculature (e.g., thrombus or fibrin sheath at a tip of the catheter, vein collapse, valves, etc.) to create a clear pathway for fluid flow. In some embodiments, the instrument 12 may reduce or remove occlusions, improving patency of the catheter for medication and fluid delivery, as well as blood acquisition, during a dwell time of the catheter.

In some embodiments, the instrument 12 may include a guidewire, a probe, a guidewire or a probe with one or more sensors, or another suitable instrument. In some embodiments, the sensors may be used for patient or device monitoring and may include sensors measuring pressure, temperature, pH, blood chemistry, oxygen saturation, flow rate, or another physiological property.
In some embodiments, the catheter may include a peripheral IV catheter, a peripherally-inserted central catheter, or a midline catheter. In some embodiments, the catheter through which the instrument 12 may be delivered may have been previously inserted into vasculature of a patient and may be dwelling within the vasculature when the instrument 12 is advanced through the catheter.

In some embodiments, the instrument 12 may be disposed within a housing 14, which may be configured to protect the instrument 12 from damage and/or contamination from a surrounding external environment. In some embodiments, the housing 14 may be rigid or semi-rigid. In some embodiments, the housing 14 may be made of one or more of stainless steel, aluminum, polycarbonate, metal, ceramic, plastic, and another suitable material. In some embodiments, the housing 14 includes a proximal end 16, a distal end 18, and a slot 20. In some embodiments, the slot 20 may extend parallel to a longitudinal axis of the housing 14.

In some embodiments, the vascular access device 10 includes an advancement element 22, which may extend through the slot 20 and may be configured to move linearly along the slot 20 between a retracted position illustrated, for example, in Figure 1A, and an advanced position. In some embodiments, the clinician may pinch or grasp the advancement element 22 to move the advancement element 22 between the retracted position and the advanced position.

In some embodiments, the distal end 18 of the housing 14 may include a connector 24. In some embodiments, the connector 24 may include opposing lever arms 26a,26b. In some embodiments, distal ends of the opposing lever arms 26a,26b may be configured to move apart from each other in response to pressure applied to proximal ends of the opposing lever arms 26a,26b. In some embodiments, in response to removal of the pressure applied to the proximal ends of the opposing lever arms 26a,26b, the distal ends may move closer to each other and clasp a portion of the catheter assembly, such as a needleless connector, another connector, or a proximal end of a catheter adapter, for example. In some embodiments, the connector 24 may include a blunt cannula or male luer configured to insert into the portion of the catheter assembly.

In some embodiments, the connector 24 may include any suitable connector. For example, the connector 24 may include a threaded male luer, a slip male luer, a threaded male luer with a spin lock, a threaded male luer with a removable blunt cannula snap connection, a slip male luer with a removable blunt cannula snap connection, or another suitable connector. In some embodiments, the connector 24 may include one or more bond pockets, which may each be configured to receive an extension tube. In some embodiments, the connector 24 may be monolithically formed as a single unit with a body of the housing 14 that includes the slot 20.

In some embodiments, the instrument 12 may include a first end 28 and a second end 30. In some embodiments, movement of the advancement element 22 from the retracted position to the advanced position may cause the second end 30 of the instrument 12 to be advanced beyond the distal end 18 of the housing 14. In some embodiments, moving the advancement element 22 to the advanced position may introduce the instrument 12 into the catheter assembly and/or through the catheter. In some embodiments, in response to the instrument 12 being introduced into the catheter assembly and/or through the catheter, the instrument 12 may access a fluid pathway of the catheter assembly and/or the vasculature of a patient.

In some embodiments, the catheter of the catheter assembly with significant dwelling time within the vasculature may be susceptible to narrowing, collapse, kinking, blockage by debris (e.g., fibrin or platelet clots), and adhering of a tip of the catheter to the vasculature. Thus, blood withdrawal using the catheter may be difficult. In some embodiments, the instrument 12 may have a diameter less than a diameter of the catheter of the catheter assembly to provide access to the vasculature of the patient without any additional needle sticks. In some embodiments, the instrument 12 may clear the pathway for collecting a blood sample. Thus, in some embodiments, the vascular access device 10 may be used for needle-free blood collection and/or fluid infusion.

In some embodiments, an extension tube 32 may be coupled to the vascular access device 10, and the extension tube 32 may be used for blood collection and/or fluid infusion. In some embodiments, the extension tube 32 may extend from a port 34 of the housing 14. In some embodiments, a fluid seal 36 may be within the housing 14 to enable the instrument 12 to advance and/or retract while maintaining a closed fluid path. In some embodiments, the instrument 12 may be configured to extend through the fluid seal 36. In some embodiments, the fluid seal 36 may be disposed proximal to the port 34 and distal to the advancement element 22 in the advanced position. In some embodiments, the fluid seal 36 may include silicone, rubber, an elastomer, or another suitable material. In some embodiments, the fluid seal 36 may include an aperture, slit, or the like to accommodate the instrument 12 therethrough.

In some embodiments, a proximal end of the extension tube 32 may be coupled to a blood collection device 38. For example, the proximal end of the extension tube 32 may be integrated with a connector 40, which may be coupled to the blood collection device 38. In some embodiments, a needleless connector may be disposed between the connector 40 and the blood collection device 38. In some embodiments, the connector 40 and/or the port 34 may be coupled to an IV line or another fluidic connection.

In some embodiments, an inner surface 42 of the housing 14 includes a groove and may include more than one groove. For example, the inner surface 42 may include a first groove 44 and/or a second groove 46. In some embodiments, the first groove 44 and/or the second groove 46 may be disposed within the housing 14 between the proximal end 16 and the distal end 18. In some embodiments, the instrument 12 may be disposed within the first groove 44 and/or the second groove 46. In some embodiments, the first groove 44 and/or the second groove 46 may include a support wall 48, another support wall 50 opposite the support wall, and a bottom 52 extending between the support wall 48 and the other support wall 50. In some embodiments, the first groove 44 and/or the second groove 46 may be open opposite the bottom 52. In some embodiments, the first groove 44 and/or the second groove 46 may be linear and/or configured to guide the instrument 12 as the instrument 12 is advanced distally and/or retracted proximally.

In some embodiments, the advancement element 22 may include an arc-shaped channel 54, which may be U-shaped. In some embodiments, the instrument 12 may extend and move through the arc-shaped channel 54. In some embodiments, the first end 28 of the instrument 12 may be fixed. In some embodiments, the first end 28 of the instrument may be fixed within the housing 14. In some embodiments, in response to movement of the advancement element 22 a first distance, the second end of the instrument 12 may be configured to advance distally a second distance. In some embodiments, the second distance may be twice the first distance. In some embodiments, the second distance may be more than twice the first distance. In these and other embodiments, the instrument 12 may extend through multiple U-shapes or other arc-shapes.

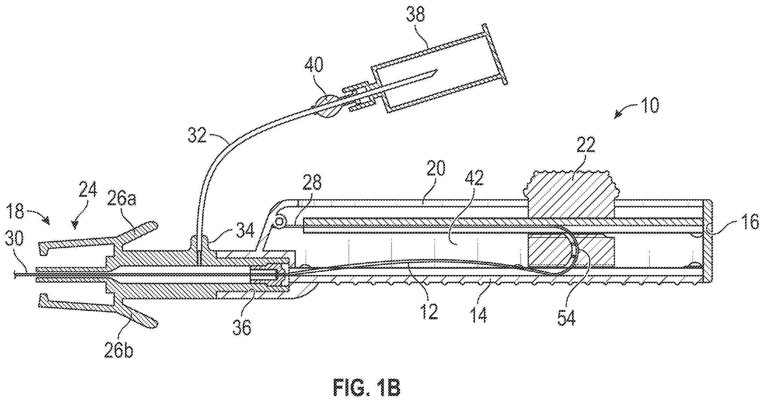

In some embodiments, because the first groove 44 and/or the second groove 46 are open opposite the bottom 52, the instrument 12 may tend to buckle in response to the advancement element 22 being advanced distally, as illustrated, for example, in Figure 1B.

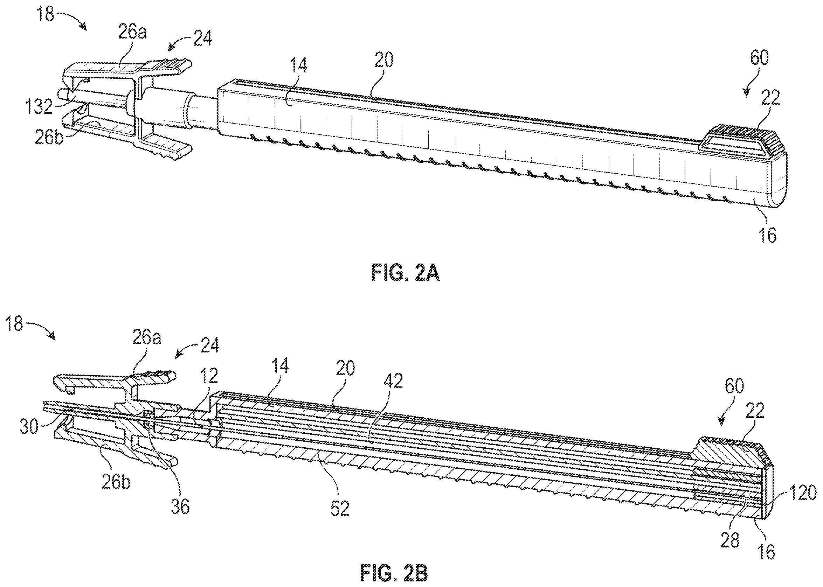

Referring now to Figures 2A-2B, another vascular access device 60 is illustrated, according to some embodiments. In some embodiments, the vascular access device 60 may be similar or identical to the vascular access device 10 in terms of one or more features and/or operation. In some embodiments, the vascular access device 60 may advance and/or retract the instrument 12 in a generally linear fashion, similar to the vascular access device 10. In some embodiments, the first end 28 may be coupled or fixed to the advancement element 22. In some embodiments, in response to movement of the advancement element 22 the first distance, the second end of the instrument 12 may be configured to advance distally a same distance as the first distance. In these embodiments, the housing 14 may include the first groove 44 and may not include the second groove 46. In some embodiments, because the first groove 44 is open opposite the bottom 52, the instrument 12 may tend to buckle in response to the advancement element 22 being advanced distally through the fluid seal 36.

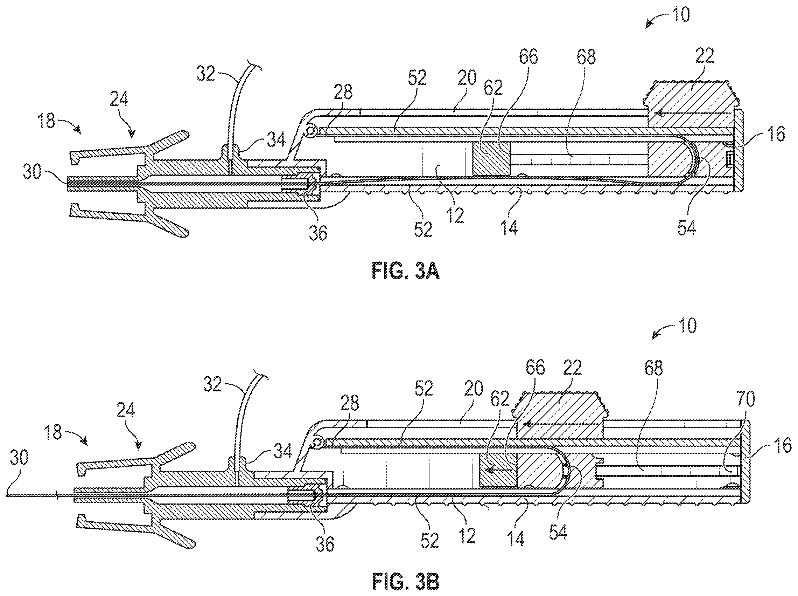

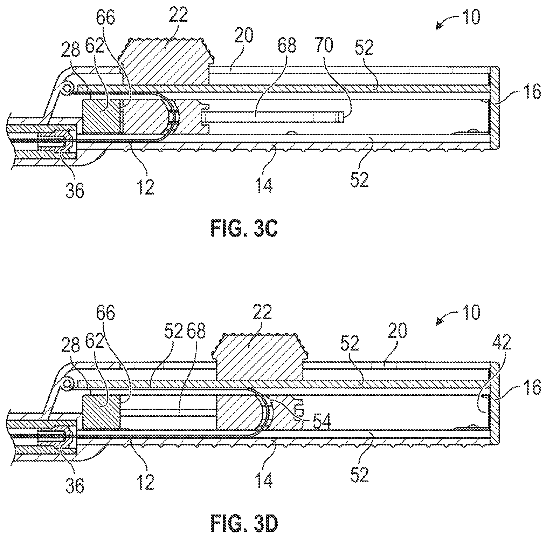

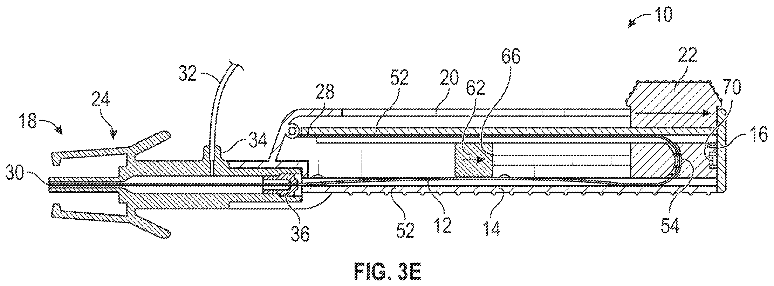

It is understood that in some embodiments, a support feature 62 of one or more of Figures 3-12 may be disposed within the vascular access device 60. For example, the spring 72 of may cover and/or be disposed within the first groove 44 of the vascular access device 60. As another example, the accordion shape 84 may be disposed within the first groove 44. As yet another example, the elastomeric discs 86 may be disposed within the first groove 44. As yet another example, the first telescoping member 88a, the strip 90, the covers 92, or the tabs 102 may cover the first groove 44. In some embodiments, the tube 98 may be disposed within the first groove 44.

Referring now to Figure 3, in some embodiments, the support feature 62 is configured to move distally in response to movement of the advancement element 22 from the retracted position, illustrated, for example, in Figure 3A, to the advanced position, illustrated, for example, in Figure 3C. In some embodiments, in response to movement of the advancement element 22 from the retracted position to the advanced position, the advancement element 22 may contact a proximal end 66 of the support feature 62 and push the support feature 62 distally along the first groove 44, as illustrated, for example, in Figure 3B.

In some embodiments, the support feature 62 may include a slider configured to slide along the housing 14. In some embodiments, the support feature 62 may be held in place within the housing 14 with a friction, interference, or snap fit. In some embodiments, when the advancement element 22 is in the retracted position, the support feature 62 may be generally in a middle of the housing or a midpoint of the first groove 44 and/or the second groove 46, which may otherwise be where buckling of the instrument 12 may tend to occur.

In some embodiments, an arm 68 may extend from the proximal end 66 of the support feature 62. In some embodiments, the arm 68 may extend through an opening in the advancement element 22. In some embodiments, in response to movement of the advancement element 22 from the retracted position to the advanced position, the advancement element 22 may slide distally along the arm 68 until the advancement element 22 contacts the proximal end 66 of the support feature 62.

In some embodiments, in response to movement of the advancement element 22 from the advanced position to the retracted position, the advancement element 22 may pull the arm 68 and the support feature 62 proximally. In some embodiments, a proximal end of the arm may include a hook 70. In some embodiments, in response in response to movement of the advancement element 22 from the advanced position to the retracted position, the advancement element 22 may catch on the hook 70 and pull the arm 68 and the support feature 62 proximally. In some embodiments, the hook 70 may include a protrusion having an outer diameter that is greater than an inner diameter of a portion of the advancement element 22, which may cause the hook 70 to catch on the advancement element 22. In some embodiments, the hook 70 may include an "L" shape. In some embodiments, the hook 70 may be coupled to the proximal end 16 of the housing 14 when the advancement element 22 is in the retracted position, which may resist movement of the support feature 62. In some embodiments, the hook 70 may be uncoupled from the proximal end 16 of the housing in response to distal movement of the advancement element 22 that pushes the support feature 62 distally.

In some embodiments, the arm 68 may be rigid, which may facilitate movement of the advancement element 22 along the arm 68. In some embodiments, the arm 68 may include a flexible tether, which may be constructed of string, ribbon, tape, or another flexible material. In these embodiments, the support feature 62 may be held in place within the housing 14 with a friction, interference, or snap fit, and may not include the hook 70.

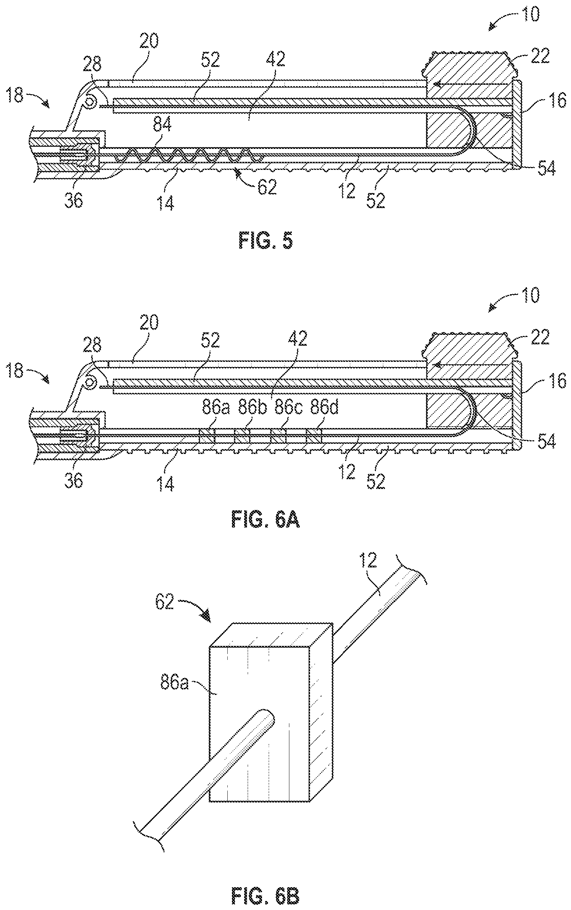

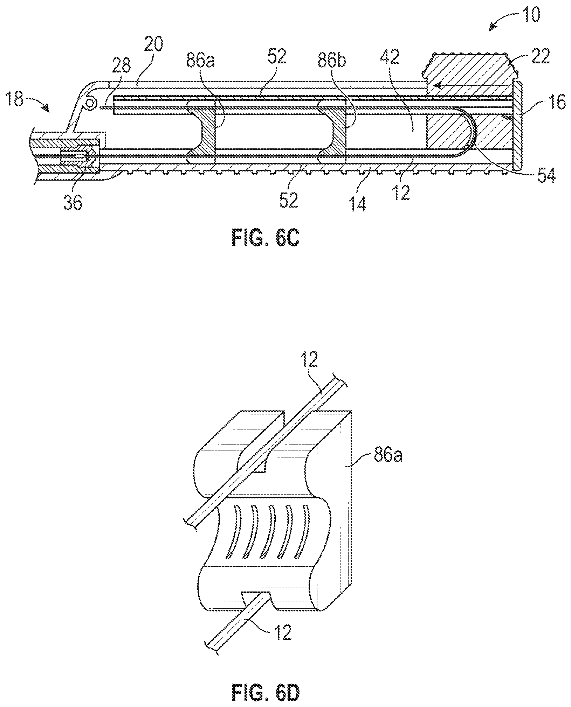

In some embodiments, the support feature 62 may cover the first groove 44 and/or the second groove 46. In some embodiments, the support feature 62 may be disposed on top of the first groove 44 and/or the second groove 46, as illustrated, for example, in Figures 3A-3E. In these and other embodiments, the support feature 62 may sit on the support wall 48 and/or the other support wall 50 (see, for example, Figure 1D). In some embodiments, the support feature 62 may extend into the first groove 44 and/or the second groove 46, which may act as a guide for the support feature 62. In these and other embodiments, the support feature 62 may be similar to one of the elastomeric discs 86 described with respect to Figures 6C-6D and may include one or more features of the elastomeric discs 86.

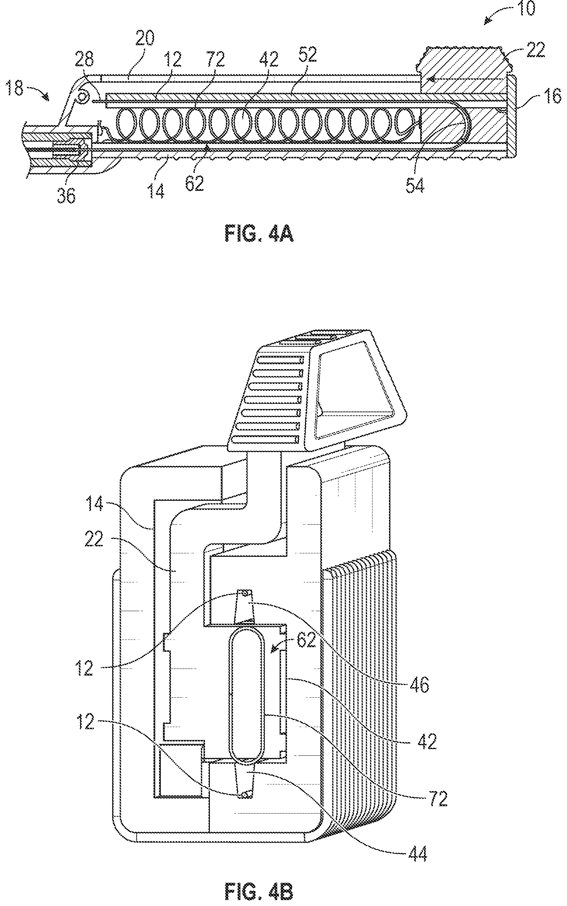

Referring now to Figures 4A-4B, in some embodiments, the support feature 62 may include a spring 72. In some embodiments, in response to movement of the advancement element 22 from the retracted position to the advanced position, the spring 72 may compress in a distal direction. In some embodiments, the spring 72 may include multiple coils. In some embodiments, in response to movement of the advancement element 22 in the distal direction, the spring 72 may be configured to contact the instrument 12 to prevent the instrument 12 from buckling or leaving the first groove 44 and/or the second groove 46. In some embodiments, the spring 72 may be helical or round. In some embodiments, the spring 72 may be oblong, as illustrated, for example, in Figure 4B. In some embodiments, the spring 72 may include a metal wire or another suitable material.

In some embodiments, the spring 72 may cover the first groove 44 and/or the second groove 46, which may facilitate retainment of the instrument 12 within the first groove 44 and/or the second groove 46. In some embodiments, the spring 72 may be disposed on top of the first groove 44 and/or the second groove 46, as illustrated, for example, in Figures 4A-4B. In these embodiments, the spring 72 may sit on or contact the support wall 48 and/or the other support wall 50 of the first groove 44 (see, for example, Figure 1D). In some embodiments, when the advancement element 22 is in the retracted position, the spring 72 may be generally in a middle of the housing 14 or a midpoint of the first groove 44 and/or the second groove 46, which may otherwise be where buckling of the instrument 12 may tend to occur.

In some embodiments, the spring 72 may extend along an entire length of the first groove 44 and/or the second groove 46. In some embodiments, the spring 72 may not extend along an entire length of the first groove 44 and/or the second groove 46. In some embodiments, a proximal end of the spring 72 may be spaced apart from the advancement element 22. In some embodiments, a distal end of the spring 72 may be spaced apart and/or proximal to the first end 28 of the instrument 12.

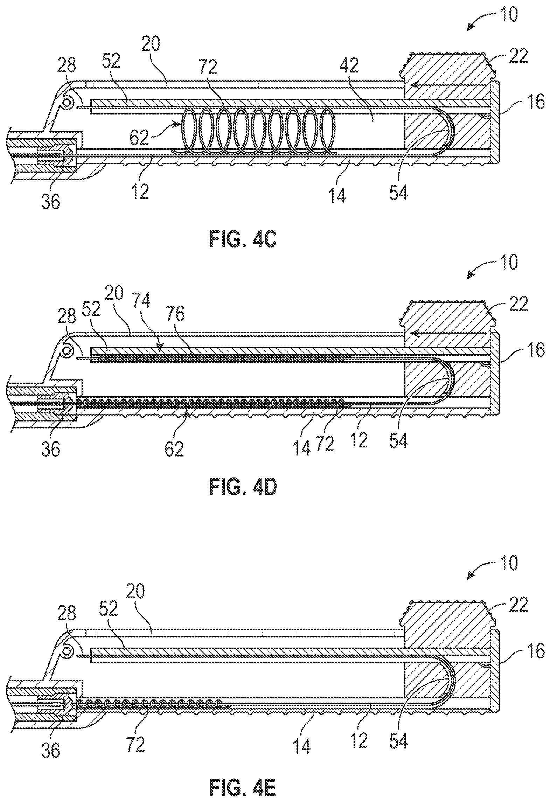

In some embodiments, a proximal end of the spring 72 may be coupled to the advancement element 22 and/or a distal end of the spring 72 may be coupled to the housing 14. Thus, in some embodiments, one or more ends of the spring 72 may be fixed. Referring now to Figure 4C, in other embodiments, the spring 72 may be freestanding or not fixed within the housing 14. In further detail, in some embodiments, the proximal end of the spring 72 may not be coupled to the advancement element 22 and the distal end of the spring 72 may not be coupled to the housing 14.

As illustrated in Figure 4C, in some embodiments, the spring 72 may extend into the first groove 44 and/or the second groove 46, which may act as a guide for the spring 72. In some embodiments, the spring 72 may be stiffer in a direction of instrument buckling than in a proximal-distal direction, which may facilitate compression of the spring 72 in the distal direction while supporting the instrument 12.

Referring now to Figure 4D, in some embodiments, the vascular access device 10 may include the support feature 62 in the first groove 44 and/or another support feature 74 in the second groove 46. In some embodiments, the other support feature 74 may be similar or identical to the support feature 62 in terms of one or more features and/or operation.

In some embodiments, the support feature 62 may include the spring 72 and/or the other support feature 74 may include another spring 76. In some embodiments, the spring 72 and/or the other spring 76 may surround the instrument 12. In these and other embodiments, the spring 72 and/or the other spring 76 may be spaced apart from the instrument 12. In some embodiments, in response to movement of the advancement element 22 from the retracted position to the advanced position, the spring 72 and the other spring 76 may compress in the distal direction. Referring now to Figure 4E, in some embodiments, the spring 72 may be disposed in the first groove 44, and the second groove 46 may not include the other spring 76.

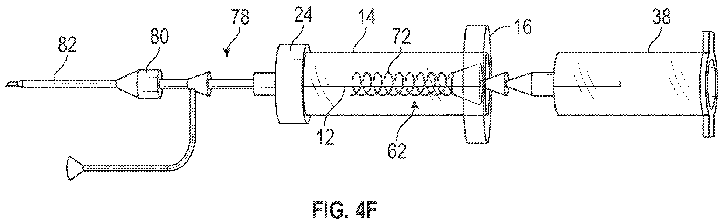

Referring now to Figure 4F, in some embodiments, the housing 14 may be collapsible in the distal direction to advance the instrument 12 in the distal direction. In some embodiments, the instrument 12 may be coupled to a portion of the housing 14, such as, for example, the proximal end 16. In some embodiments, in response to the housing 14 collapsing in the distal direction, the spring 72 may be compressed and the proximal end 16 of the housing 14 may be coupled to the connector 24. In some embodiments, the connector 24 may include a female luer that may receive a male luer of the proximal end 16 of the housing 14 in response to the housing 14 collapsing in the distal direction. In some embodiments, the instrument 12 may extend through the spring 72.

In some embodiments, the connector 24 may be coupled to a catheter assembly 78, which may include a catheter adapter 80 and a catheter 82 extending distally from the catheter 82. In some embodiments, the connector 24 may be coupled to the catheter assembly 78 via one or more extension tubes and/or one or more other connectors. In some embodiments, the blood collection device 38 may be coupled to a proximal end of the instrument 12. In these and other embodiments, the instrument 12 may include a tube through which blood may flow in response to insertion of the catheter assembly 78 into the vasculature of the patient.

Referring now to Figure 5, in some embodiments, the support feature 62 may include an accordion shape 84. In some embodiments, in response to movement of the advancement element 22 from the retracted position to the advanced position, the accordion shape 84 may compress in the distal direction. In some embodiments, the instrument 12 may extend through the accordion shape 84, such as through an opening in a middle of the accordion shape 84, for example. In some embodiments, the second groove 46 may include an accordion shape therein similar or identical to the accordion shape 84.

Referring now to Figures 6A-6D, in some embodiments, the support feature 62 may include one or more elastomeric discs 86 disposed within the first groove 44. For example, a first elastomeric disc 86a, a second elastomeric disc 86b, a third elastomeric disc 86c, and a fourth elastomeric disc 86d (collectively referred to in the present disclosure as "elastomeric disc 86") may be disposed within the first groove 44. In some embodiments, the vascular access device 10 may include between one and ten of the elastomeric discs 86 within the first groove 44. In some embodiments, the vascular access device 10 may include more than ten of the elastomeric discs 86 within the first groove 44.

In some embodiments, the elastomeric discs 86 may be spaced apart and/or evenly spaced with respect to each other. In some embodiments, in response to movement of the advancement element 22 from the retracted position to the advanced position, each of the elastomeric discs 86 may slide distally within the first groove 44 and/or may contact each other to form a stack. In some embodiments, the second groove 46 may include one or more elastomeric discs therein similar or identical to the elastomeric discs 86 in terms of one or more features and/or operation.

As illustrated in Figures 6C-6D, in some embodiments, the elastomeric discs 86 may extend between the first groove 44 and the second groove 46. In some embodiments, each of the elastomeric discs 86 may be disposed within the first groove 44 and/or the second groove 46. In some embodiments, each of the elastomeric discs 86 may include a first slot and/or a second slot through which the instrument 12 extends. In some embodiments, the first slot may be disposed within the first groove 44 and/or the second slot may be disposed within the second groove 46.

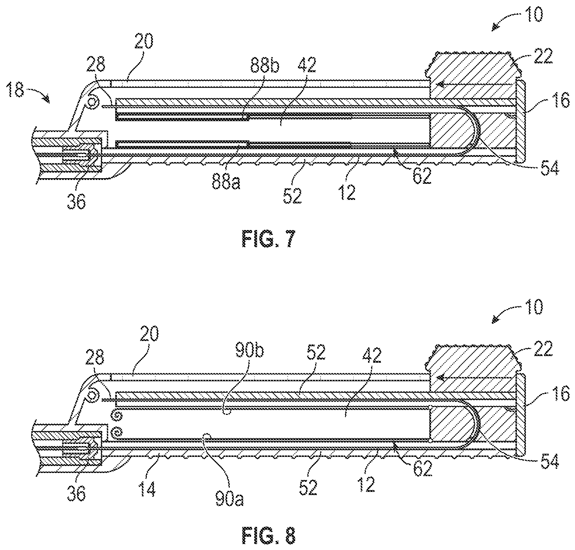

Referring now to Figure 7, the support feature 62 may include a first telescoping member 88a. In some embodiments, the vascular access device 10 may include the first telescoping member 88a covering the first groove 44 and/or a second telescoping member 88b covering the second groove 46. In some embodiments, in response to movement of the advancement element 22 from the retracted position to the advanced position, the first telescoping member 88a and/or the second telescoping member 88b may collapse distally due to contact with the advancement element 22. In further detail, in some embodiments, the first telescoping member 88a and/or the second telescoping member 88b may include two or more pieces. In some embodiments, a most proximal one of the pieces may include an inner diameter large enough to receive one or more distal pieces configured to collapse into the most proximal one of the pieces.

In some embodiments, the first telescoping member 88a may extend along an entire length of the first groove 44 and/or the second telescoping member 88b may extend along an entire length of the second groove 46, which may reduce buckling of the instrument 12. In some embodiments, a proximal end of the first telescoping member 88a and/or a proximal end of the second telescoping member 88b may be coupled to the advancement element 22. In some embodiments, a distal end of the first telescoping member 88a and/or a distal end of the second telescoping member 88b may be coupled to the inner surface 42 of the housing 14 or configured to push against the housing 14.

In some embodiments, in response to movement of the advancement element 22 from the advanced position to the retracted position, the first telescoping member 88a and/or the second telescoping member 88b may expand proximally and/or may include catches that bias them in an expanded position, illustrated, for example, in Figure 7. In some embodiments, a shape of the first telescoping member 88a and/or the second telescoping member 88b may by cylindrical, rectangular, or any other suitable shape. In some embodiments, the first telescoping member 88a may be disposed within the first groove 44 and/or the second telescoping member 88b may be disposed within the second groove 46. In these and other embodiments, the first telescoping member 88a and/or the second telescoping member 88b may be generally flat.

Referring now to Figure 8, the support feature 62 may include a first strip 90a. In some embodiments, the vascular access device 10 may include the first strip 90a covering the first groove 44 and/or a second strip 90b covering the second groove 46. In some embodiments, in response to movement of the advancement element 22 from the retracted position to the advanced position, the first strip 90a and/or the second strip 90b may roll up.

In some embodiments, the first strip 90a and/or the second strip 90b may each include a first end and a second end. In some embodiments, the first end may be coupled to the advancement element 22, and the second end may be configured to roll up or coil in response to movement of the advancement element 22 from the retracted position to the advancement position. In some embodiments, the first end may include a proximal end of the first strip 90a and/or the second strip 90b, and the second end may include a distal end of the first strip 90a and/or the second strip 90b.

In these and other embodiments, the first end may be coupled or fixed to the advancement element 22 and/or the second end may be coupled to or fixed to the housing 14. In some embodiments, the first strip 90a and/or the second strip 90b may be configured to roll up around a pin or fixed element with respect to the housing 14. In some embodiments, the second end configured to roll up may be disposed within the advancement element 22 and/or the first end may be coupled or fixed to the housing 14. In some embodiments, the first strip 90a and/or the second strip 90b may be flat or arced across its width.

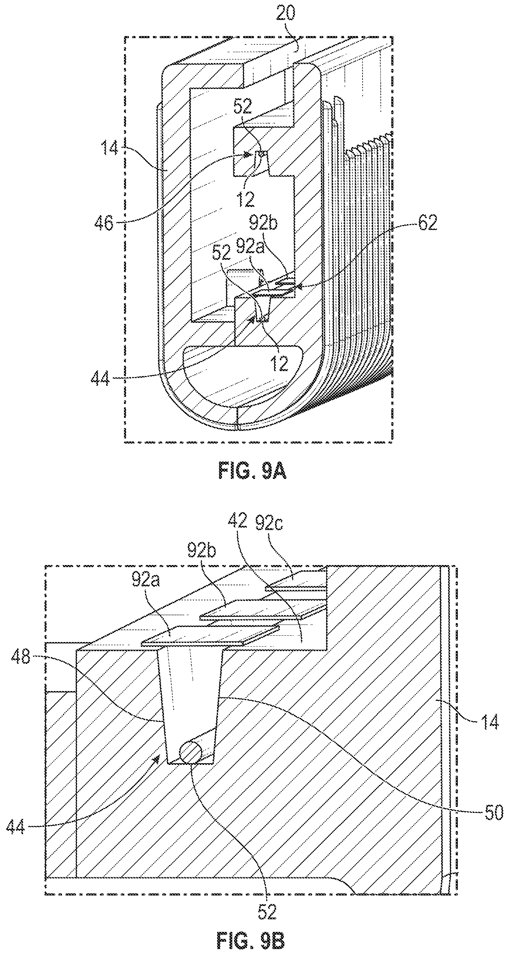

Referring now to Figures 9A-9B, in some embodiments, the support feature 62 may include one or more covers 92 extending over the first groove 44 and/or the second groove 46. Figures 9A-9C illustrate a first cover 92a, a second cover 92b, and a third cover 92c (which may be referred to collectively in the present disclosure as "covers 92"), according to some embodiments. In some embodiments, any number of covers 92 may extend over the first groove 44 and/or the second groove 46.

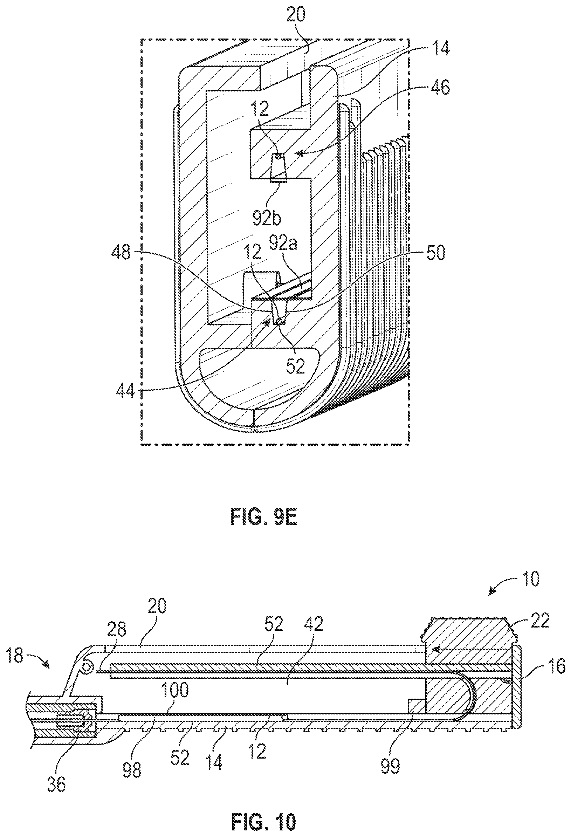

In some embodiments, a first side of a particular cover 92 and/or a second side of the particular cover 92 opposite the first side may be coupled to the housing 14. In some embodiments, the covers 92 may each include a thin film or another suitable material. In some embodiments, the covers 92 may be split or cut by the advancement element 22 in response to movement of the advancement element 22 from the retracted position to the advanced position. An example blade 99 of the advancement element 22 that may split or cut the covers 92 is illustrated in Figure 10. In some embodiments, the covers 92 may be constructed of a flexible material but stiff enough to provide support to the instrument 12 to prevent buckling. In some embodiments, the flexible material may facilitate movement of a central portion of the covers 92 or spreading of the covers 92.

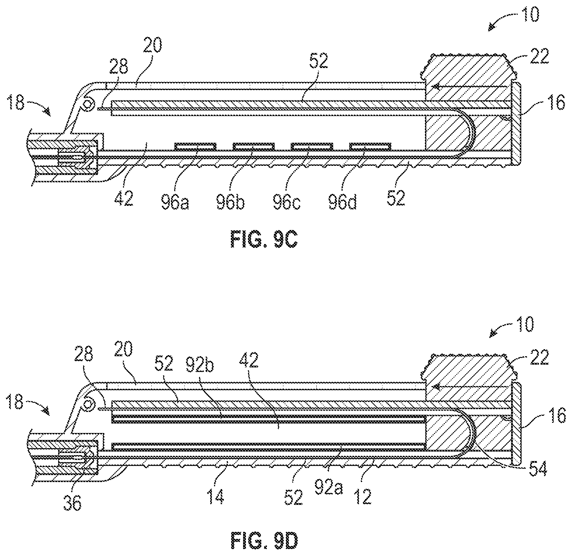

Referring now to Figures 9D-9E, in some embodiments, the vascular access device 10 may include only one of the covers 92 on the first groove 44 and/or only one of the covers 92 on the second groove 46. For example, a cover 92a may extend along a majority or entirety of a length of the first groove 44 and/or a cover 92b may extend along a majority or entirety of a length of the second groove 46.

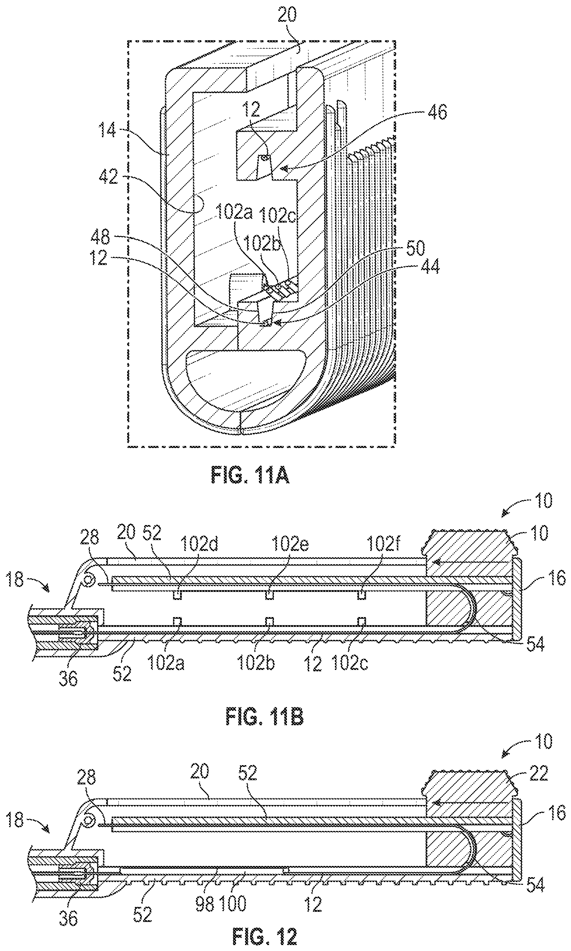

Referring now to Figures 10 and 12, in some embodiments, a tube 98 is disposed within the first groove 44. In some embodiments, a similar tube may be disposed within the second groove 96. In some embodiments, as illustrated, for example, in Figure 10, the instrument 12 extends through the tube 98. In some embodiments, the tube 98 may include a slit 100, and in response to movement of the advancement element 22 from the retracted position to the advanced position, the advancement element 22 may move distally through the slit 100. In some embodiments, the covers 92 of Figure 9 may each include a slit similar to the slit 100. As illustrated, for example, in Figure 10, in some embodiments, the advancement element 22 may include the arc-shaped channel 54, and in response to movement of the advancement element 22 from the retracted position to the advanced position, the tube 98 may slide into the arc-shaped channel 54.

In some embodiments, the tube 98 may be constructed of a flexible material but stiff enough to provide support to the instrument 12 to prevent buckling. In some embodiments, the flexible material may facilitate movement of a central portion of the tube 98 or spreading of the tube 98 to allow entry or passage of the advancement element 22.

Referring now to Figures 11A-11B, in some embodiments, the support feature 62 may include one or more tabs 102 extending over the first groove 44 and/or the second groove 46. In some embodiments, the tabs 102 may be configured to swivel and/or bend in the distal direction response to movement of the advancement element 22 from the retracted position to the advanced position and contact by the advancement element 22. In some embodiments, each of the tabs 102 may swivel or pivot around a pin coupling each of the tabs 102 to the housing 14. In some embodiments, the tabs 102 may be constructed of a flexible material to facilitate bending in distal direction and passage of the advancement element 22. In some embodiments, the tabs 102 may not move or bend in the direction of the buckling of the instrument 12 in response to passage of the advancement element 22, and therefore may support the instrument 12.

All examples and conditional language recited herein are intended for pedagogical objects to aid the reader in understanding the invention and the concepts contributed by the inventor to furthering the art and are to be construed as being without limitation to such specifically recited examples and conditions. Although embodiments of the present inventions have been described in detail, it should be understood that the various changes, substitutions, and alterations could be made hereto without departing from the scope of the invention.

## Claims

1. A vascular access device (10), comprising:
a housing (14), comprising a proximal end (16), a distal end (18), and a slot (20), wherein an inner surface (42) of the housing (14) comprises a groove (44) disposed within the housing (14) between the proximal end (16) of the housing (14) and the distal end (18) of the housing (14);
an advancement element (22) extending through the slot (20) and configured to move linearly along the slot (20) between a retracted position and an advanced position;
an instrument (12) disposed within the groove (44), the instrument (12) comprising a first end (28) and a second end (30), wherein in response to movement of the advancement element (22) from the retracted position to the advanced position, the second end (30) of the instrument (12) is advanced beyond the distal end (18) of the housing (14); and
a support feature (62) disposed on top of the groove (44) or within the groove (44) and configured to move distally in response to movement of the advancement element (22) from the retracted position to the advanced position, wherein the support feature (62) limits displacement of the instrument (12) from the groove (44), wherein the support feature (62) is positioned distally from the advancement element (22).

2. The vascular access device (10) of claim 1, wherein the groove (44) is linear.

3. The vascular access device (10) of claim 1, wherein the support feature (62) is disposed on top of the groove (44), wherein in response to movement of the advancement element (22) from the retracted position to the advanced position, the advancement element (22) contacts a proximal end (66) of the support feature (62) and pushes the support feature (62) distally along the groove (44).

4. The vascular access device (10) of claim 3, further comprising an arm (68) extending from the proximal end (66) of the support feature (62), wherein the arm (68) extends through the advancement element (22), wherein in response to movement of the advancement element (22) from the retracted position to the advanced position, the advancement element (22) slides distally along the arm (68) until the advancement element (22) contacts the proximal end (66) of the support feature (62).

5. The vascular access device (10) of claim 4, wherein in response to movement of the advancement element (22) from the advanced position to the retracted position, the advancement element (22) pulls the arm (68) and the support feature (62) proximally.

6. The vascular access device (10) of claim 5, wherein a proximal end of the arm (68) comprises a hook (70), wherein in response to movement of the advancement element (22) from the advanced position to the retracted position, the advancement element (22) catches on the hook (70) and pulls the arm (68) and the support feature (62) proximally.

7. The vascular access device (10) of claim 1, wherein the support feature (62) comprises a spring (72), wherein in response to movement of the advancement element (22) from the retracted position to the advanced position, the spring (72) compresses in a distal direction, wherein the spring (72) is preferably disposed within the groove (44) and the instrument (12) extends through the spring (72), or the spring (72) is disposed on top of the groove (44).

8. The vascular access device (10) of claim 1, wherein the advancement element (22) comprises an arc-shaped channel (54), wherein the instrument (12) extends through the arc-shaped channel (54), wherein the first end (28) of the instrument (12) is fixed, wherein in response to movement of the advancement element (22) a first distance, the second end (30) of the instrument (12) is configured to advance distally a second distance, wherein the second distance is at least twice the first distance, wherein the groove (44) is a first groove (44), wherein the inner surface (42) of the housing (14) comprises a second groove (46) between the proximal end (16) of the housing (14) and the distal end (18) of the housing (14) and generally parallel to the first groove (44), wherein the instrument (12) extends through the second groove (46).

9. The vascular access device (10) of claim 8, wherein the support feature (62) is disposed between the first groove (44) and the second groove (46) and limits displacement of the instrument (12) from the second groove (46) or wherein the support feature (62) is disposed within the first groove (44), further comprising another support feature (74) disposed within the second groove (46), wherein the instrument (12) extends through the support feature (62) and the other support feature (74).

10. The vascular access device (10) of claim 9, wherein the support feature (62) and the other support feature (74) each comprise a spring (72, 76).

11. The vascular access device (10) of claim 1, wherein the support feature (62) comprises an accordion shape (84), wherein in response to movement of the advancement element (22) from the retracted position to the advanced position, the accordion shape (84) compresses in a distal direction or wherein the support feature (62) comprises a plurality of elastomeric discs (86) disposed within the groove (44), wherein in response to movement of the advancement element (22) from the retracted position to the advanced position, each of the plurality of elastomeric discs (86) slides distally within the groove (44).

12. The vascular access device (10) of claim 1, wherein the support feature (62) comprises a telescoping member (88a, 88b) and/or wherein the support feature (62) comprises a strip (90a, 90b), wherein the strip (90a, 90b) comprises a first end and a second end, wherein the first end is coupled to the advancement element (22) and the second end is configured to roll up in response to movement of the advancement element (22) from the retracted position to the advancement position.

13. The vascular access device (10) of claim 1, wherein the support feature (62) comprises a cover (92) extending over the groove (44), wherein a first side of the cover (92) or a second side of the cover (92) opposite the first side of the cover (92) is coupled to the housing (14) or wherein a first side of the cover (92) and the second side of the cover (92) opposite the first side are coupled to the housing (14), wherein the cover (92) comprises a slit down a middle of the cover or the advancement element (22) comprises a blade (99) that cuts through the cover (92) in response to movement of the advancement element (22) from the retracted position to the advanced position.

14. The vascular access device (10) of claim 1, wherein the support feature (62) comprises a plurality of tabs (102) extending over the groove (44) and configured to swivel or bend in response to movement of the advancement element (22) from the retracted position to the advanced position and contact by the advancement element (22).

15. A vascular access device (10), comprising:
a housing (14), comprising a proximal end (16), a distal end (18), and a slot (20), wherein an inner surface (42) of the housing (14) comprises a groove (44) disposed within the housing (14) between the proximal end (16) of the housing (14) and the distal end (18) of the housing (14);
an advancement element (22) extending through the slot (20) and configured to move linearly along the slot (20) between a retracted position and an advanced position;
an instrument (12) disposed within the groove (44), the instrument (12) comprising a first end (28) and a second end (30), wherein in response to movement of the advancement element (22) from the retracted position to the advanced position, the second end (30) of the instrument (12) is advanced beyond the distal end (18) of the housing (14); and
a tube (98) disposed within the groove (44), wherein the instrument (12) extends through the tube (98), wherein:
the tube (98) comprises a slit (100) and in response to movement of the advancement element (22) from the retracted position to the advanced position, the advancement element (22) moves distally through the slit (100); or
the advancement element (22) comprises an arc-shaped channel (54) and in response to movement of the advancement element (22) from the retracted position to the advanced position, the tube (98) slides proximally into the arc-shaped channel (54).

## Patentansprüche

1. Gefäßzugangsvorrichtung (10), die aufweist:
ein Gehäuse (14), das ein proximales Ende (16), ein distales Ende (18) und einen Spalt (20) aufweist, wobei eine Innenfläche (42) des Gehäuses (14) eine Nut (44) aufweist, die innerhalb des Gehäuses (14) zwischen dem proximalen Ende (16) des Gehäuses (14) und dem distalen Ende (18) des Gehäuses (14) angeordnet ist;
ein Vorschubelement (22), das sich durch den Spalt (20) erstreckt und so ausgebildet ist, dass es sich linear entlang des Spalts (20) zwischen einer eingefahrenen Position und einer ausgefahrenen Position bewegen kann;
ein Instrument (12), das in der Nut (44) angeordnet ist, wobei das Instrument (12) ein erstes Ende (28) und ein zweites Ende (30) aufweist, wobei als Reaktion auf eine Bewegung des Vorschubelements (22) von der eingefahrenen Position in die ausgefahrene Position das zweite Ende (30) des Instruments (12) über das distale Ende (18) des Gehäuses (14) hinaus vorgeschoben wird; und
ein Stützelement (62), das auf der Nut (44) oder innerhalb der Nut (44) angeordnet und so ausgebildet ist, dass es sich als Reaktion auf eine Bewegung des Vorschubelements (22) von der eingefahrenen Position in die ausgefahrene Position distal bewegt, wobei das Stützelement (62) die Verschiebung des Instruments (12) aus der Nut (44) heraus begrenzt, wobei das Stützelement (62) distal vom Vorschubelement (22) positioniert ist.

2. Gefäßzugangsvorrichtung (10) nach Anspruch 1, wobei die Nut (44) linear ist.

3. Gefäßzugangsvorrichtung (10) nach Anspruch 1, wobei das Stützelement (62) auf der Nut (44) angeordnet ist, wobei als Reaktion auf eine Bewegung des Vorschubelements (22) von der eingefahrenen Position in die ausgefahrene Position das Vorschubelement (22) ein proximales Ende (66) des Stützelements (62) berührt und das Stützelement (62) entlang der Nut (44) distal verschiebt.

4. Gefäßzugangsvorrichtung (10) nach Anspruch 3, die ferner einen Arm (68) aufweist, der sich ausgehend vom proximalen Ende (66) des Stützelements (62) erstreckt, wobei sich der Arm (68) durch das Vorschubelement (22) erstreckt, wobei als Reaktion auf eine Bewegung des Vorschubelements (22) von der eingefahrenen Position in die ausgefahrene Position das Vorschubelement (22) distal entlang des Arms (68) gleitet, bis das Vorschubelement (22) das proximale Ende (66) des Stützelements (62) berührt.

5. Gefäßzugangsvorrichtung (10) nach Anspruch 4, wobei das Vorschubelement (22) als Reaktion auf eine Bewegung des Vorschubelements (22) von der ausgefahrenen Position in die eingefahrene Position den Arm (68) und das Stützelement (62) proximal zieht.

6. Gefäßzugangsvorrichtung (10) nach Anspruch 5, wobei ein proximales Endes des Arms (68) einen Haken (70) aufweist, wobei das Vorschubelement (22) als Reaktion auf eine Bewegung des Vorschubelements (22) von der ausgefahrenen Position in die eingefahrene Position am Haken (70) einrastet und den Arm (68) und das Stützelement (62) proximal zieht.

7. Gefäßzugangsvorrichtung (10) nach Anspruch 1, wobei das Stützelement (62) eine Feder (72) aufweist, wobei die Feder (72) als Reaktion auf eine Bewegung des Vorschubelements (22) von der eingefahrenen Position in die ausgefahrene Position in distaler Richtung zusammengedrückt wird, wobei die Feder (72) vorzugsweise innerhalb der Nut (44) angeordnet ist und sich das Instrument (12) durch die Feder (72) erstreckt, oder die Feder (72) auf der Nut (44) angeordnet ist.

8. Gefäßzugangsvorrichtung (10) nach Anspruch 1, wobei das Vorschubelement (22) einen bogenförmigen Kanal (54) aufweist, wobei sich das Instrument (12) durch den bogenförmigen Kanal (54) erstreckt, wobei das erste Ende (28) des Instruments (12) fixiert ist, wobei als Reaktion auf eine Bewegung des Vorschubelements (22) um eine erste Strecke das zweite Ende (30) des Instruments (12) so ausgebildet ist, dass es sich um eine zweite Strecke distal verschiebt, wobei die zweite Strecke mindestens doppelt so groß ist wie die erste Strecke, wobei die Nut (44) eine erste Nut (44) ist, wobei die Innenfläche (42) des Gehäuses (14) eine zweite Nut (46) zwischen dem proximalen Ende (16) des Gehäuses (14) und dem distalen Ende (18) des Gehäuses (14) aufweist, die im Wesentlichen parallel zur ersten Nut (44) verläuft, wobei sich das Instrument (12) durch die zweite Nut (46) erstreckt.

9. Gefäßzugangsvorrichtung (10) nach Anspruch 8, wobei das Stützelement (62) zwischen der ersten Nut (44) und der zweiten Nut (46) angeordnet ist und die Verschiebung des Instruments (12) aus der zweiten Nut (46) begrenzt oder wobei das Stützelement (62) innerhalb der ersten Nut (44) angeordnet ist, ferner aufweisend ein weiteres Stützelement (74), das innerhalb der zweiten Nut (46) angeordnet ist, wobei sich das Instrument (12) durch das Stützelement (62) und das weitere Stützelement (74) erstreckt.

10. Gefäßzugangsvorrichtung (10) nach Anspruch 9, wobei das Stützelement (62) und das andere Stützelement (74) jeweils eine Feder (72, 76) aufweisen.

11. Gefäßzugangsvorrichtung (10) nach Anspruch 1, wobei das Stützelement (62) eine Ziehharmonikaform (84) aufweist, wobei sich die Ziehharmonikaform (84) als Reaktion auf eine Bewegung des Vorschubelements (22) von der eingefahrenen Position in die ausgefahrene Position in distaler Richtung zusammenzieht, oder wobei das Stützelement (62) eine Vielzahl von Elastomerscheiben (86) aufweist, die innerhalb der Nut (44) angeordnet sind, wobei als Reaktion auf eine Bewegung des Vorschubelements (22) von der eingefahrenen Position in die ausgefahrene Position jede der Vielzahl von Elastomerscheiben (86) distal innerhalb der Nut (44) gleitet.

12. Gefäßzugangsvorrichtung (10) nach Anspruch 1, wobei das Stützelement (62) ein Teleskopelement (88a, 88b) aufweist und/oder wobei das Stützelement (62) einen Streifen (90a, 90b) aufweist, wobei der Streifen (90a, 90b) ein erstes Ende und ein zweites Ende aufweist, wobei das erste Ende mit dem Vorschubelement (22) verbunden ist und das zweite Ende so ausgebildet ist, dass es sich als Reaktion auf eine Bewegung des Vorschubelements (22) von der eingefahrenen Position in die Vorschubposition aufrollt.

13. Gefäßzugangsvorrichtung (10) nach Anspruch 1, wobei das Stützelement (62) eine Abdeckung (92) aufweist, die sich über der Nut (44) erstreckt, wobei eine erste Seite der Abdeckung (92) oder eine zweite Seite der Abdeckung (92) entgegengesetzt zur ersten Seite der Abdeckung (92) mit dem Gehäuse (14) verbunden ist oder wobei eine erste Seite der Abdeckung (92) und die zweite Seite der Abdeckung (92) entgegengesetzt zur ersten Seite mit dem Gehäuse (14) verbunden sind, wobei die Abdeckung (92) einen Schlitz in der Mitte der Abdeckung aufweist oder das Vorschubelement (22) eine Klinge (99) aufweist, welche die Abdeckung (92) als Reaktion auf eine Bewegung des Vorschubelements (22) von der eingefahrenen Position in die ausgefahrene Position durchschneidet.

14. Gefäßzugangsvorrichtung (10) nach Anspruch 1, wobei das Stützelement (62) eine Vielzahl von Laschen (102) aufweist, die sich über die Nut (44) erstrecken und so ausgebildet sind, dass sie als Reaktion auf eine Bewegung des Vorschubelements (22) von der eingefahrenen Position in die ausgefahrene Position sowie auf den Kontakt mit dem Vorschubelement (22) schwenken oder sich biegen.

15. Gefäßzugangsvorrichtung (10), die aufweist:
ein Gehäuse (14), das ein proximales Ende (16), ein distales Ende (18) und einen Spalt (20) aufweist, wobei eine Innenfläche (42) des Gehäuses (14) eine Nut (44) aufweist, die innerhalb des Gehäuses (14) zwischen dem proximalen Ende (16) des Gehäuses (14) und dem distalen Ende (18) des Gehäuses (14) angeordnet ist;
ein Vorschubelement (22), das sich durch den Spalt (20) erstreckt und so ausgebildet ist, dass es sich linear entlang des Spalts (20) zwischen einer eingefahrenen Position und einer ausgefahrenen Position bewegen kann;
ein Instrument (12), das in der Nut (44) angeordnet ist, wobei das Instrument (12) ein erstes Ende (28) und ein zweites Ende (30) aufweist, wobei als Reaktion auf eine Bewegung des Vorschubelements (22) von der eingefahrenen Position in die ausgefahrene Position das zweite Ende (30) des Instruments (12) über das distale Ende (18) des Gehäuses (14) hinaus vorgeschoben wird; und
ein Rohr (98), das in der Nut (44) angeordnet ist, wobei sich das Instrument (12) durch das Rohr (98) erstreckt, wobei:
das Rohr (98) einen Schlitz (100) aufweist und sich das Vorschubelement (22) als Reaktion auf eine Bewegung des Vorschubelements (22) von der eingefahrenen Position in die ausgefahrene Position distal durch den Schlitz (100) bewegt; oder
das Vorschubelement (22) einen bogenförmigen Kanal (54) aufweist und das Rohr (98) als Reaktion auf eine Bewegung des Vorschubelements (22) von der eingefahrenen Position in die ausgefahrene Position proximal in den bogenförmigen Kanal (54) hinein gleitet.

## Revendications

1. Dispositif d'accès vasculaire (10) comprenant :
un boîtier (14), comprenant une extrémité proximale (16), une extrémité distale (18) et une fente (20), dans lequel une surface interne (42) du boîtier (14) comprend une rainure (44) disposée à l'intérieur du boîtier (14) entre l'extrémité proximale (16) du boîtier (14) et l'extrémité distale (18) du boîtier (14) ;
un élément d'avancement (22) s'étendant à travers la fente (20) et configuré pour se déplacer linéairement le long de la fente (20) entre une position rétractée et une position avancée ;
un instrument (12) disposé à l'intérieur de la rainure (44), l'instrument (12) comprenant une première extrémité (28) et une seconde extrémité (30), dans lequel, en réponse au mouvement de l'élément d'avancement (22) de la position rétractée à la position avancée, la seconde extrémité (30) de l'instrument (12) est avancée au-delà de l'extrémité distale (18) du boîtier (14) ; et
une partie de support (62) disposée sur le dessus de la rainure (44) ou à l'intérieur de la rainure (44) et configurée pour se déplacer distalement en réponse au mouvement de l'élément d'avancement (22) de la position rétractée à la position avancée, dans lequel la partie de support (62) limite le déplacement de l'instrument (12) à partir de la rainure (44), dans lequel la partie de support (62) est positionnée distalement à partir de l'élément d'avancement (22).

2. Dispositif d'accès vasculaire (10) selon la revendication 1, dans lequel la rainure (44) est linéaire.

3. Dispositif d'accès vasculaire (10) selon la revendication 1, dans lequel la partie de support (62) est disposée sur le dessus de la rainure (44), dans lequel en réponse au mouvement de l'élément d'avancement (22) de la position rétractée à la position avancée, l'élément d'avancement (22) entre en contact avec une extrémité proximale (66) de la partie de support (62) et pousse la partie de support (62) distalement le long de la rainure (44).

4. Dispositif d'accès vasculaire (10) selon la revendication 3, comprenant en outre un bras (68) s'étendant à partir de l'extrémité proximale (66) de la partie de support (62), dans lequel le bras (68) s'étend à travers l'élément d'avancement (22), dans lequel en réponse au mouvement de l'élément d'avancement (22) de la position rétractée à la position avancée, l'élément d'avancement (22) coulisse distalement le long du bras (68) jusqu'à ce que l'élément d'avancement (22) entre en contact avec l'extrémité proximale (66) de la partie de support (62).

5. Dispositif d'accès vasculaire (10) selon la revendication 4, dans lequel, en réponse au mouvement de l'élément d'avancement (22) de la position avancée à la position rétractée, l'élément d'avancement (22) tire le bras (68) et la partie de support (62) proximalement.

6. Dispositif d'accès vasculaire (10) selon la revendication 5, dans lequel une extrémité proximale du bras (68) comprend un crochet (70), dans lequel en réponse au mouvement de l'élément d'avancement (22) de la position avancée à la position rétractée, l'élément d'avancement (22) s'accroche sur le crochet (70) et tire le bras (68) et la partie de support (62) proximalement.

7. Dispositif d'accès vasculaire (10) selon la revendication 1, dans lequel la partie de support (62) comprend un ressort (72), dans lequel, en réponse au mouvement de l'élément d'avancement (22) de la position rétractée à la position avancée, le ressort (72) se comprime dans une direction distale, dans lequel le ressort (72) est de préférence disposé à l'intérieur de la rainure (44) et l'instrument (12) s'étend à travers le ressort (72), ou le ressort (72) est disposé sur le dessus de la rainure (44).

8. Dispositif d'accès vasculaire (10) selon la revendication 1, dans lequel l'élément d'avancement (22) comprend un canal en forme d'arc (54), dans lequel l'instrument (12) s'étend à travers le canal en forme d'arc (54), dans lequel la première extrémité (28) de l'instrument (12) est fixe, dans lequel en réponse à un mouvement de l'élément d'avancement (22) d'une première distance, la seconde extrémité (30) de l'instrument (12) est configurée pour avancer distalement d'une seconde distance, dans lequel la seconde distance est au moins deux fois la première distance, dans lequel la rainure (44) est une première rainure (44), dans lequel la surface interne (42) du boîtier (14) comprend une seconde rainure (46) entre l'extrémité proximale (16) du boîtier (14) et l'extrémité distale (18) du boîtier (14) et généralement parallèle à la première rainure (44), dans lequel l'instrument (12) s'étend à travers la seconde rainure (46).

9. Dispositif d'accès vasculaire (10) selon la revendication 8, dans lequel la partie de support (62) est disposée entre la première rainure (44) et la seconde rainure (46) et limite le déplacement de l'instrument (12) à partir de la seconde rainure (46) ou dans lequel la partie de support (62) est disposée à l'intérieur de la première rainure (44), comprenant en outre une autre partie de support (74) disposée à l'intérieur de la seconde rainure (46), dans lequel l'instrument (12) s'étend à travers la partie de support (62) et l'autre partie de support (74).

10. Dispositif d'accès vasculaire (10) selon la revendication 9, dans lequel la partie de support (62) et l'autre partie de support (74) comprennent chacune un ressort (72, 76).

11. Dispositif d'accès vasculaire (10) selon la revendication 1, dans lequel la partie de support (62) comprend une forme en accordéon (84), dans lequel en réponse au mouvement de l'élément d'avancement (22) de la position rétractée à la position avancée, la forme en accordéon (84) se comprime dans une direction distale ou dans lequel la partie de support (62) comprend une pluralité de disques élastomères (86) disposés à l'intérieur de la rainure (44), dans lequel en réponse au mouvement de l'élément d'avancement (22) de la position rétractée à la position avancée, chacun de la pluralité de disques élastomères (86) coulisse distalement à l'intérieur de la rainure (44).

12. Dispositif d'accès vasculaire (10) selon la revendication 1, dans lequel la partie de support (62) comprend un organe télescopique (88a, 88b) et/ou dans lequel la partie de support (62) comprend une bande (90a, 90b), dans lequel la bande (90a, 90b) comprend une première extrémité et une seconde extrémité, dans lequel la première extrémité est couplée à l'élément d'avancement (22) et la seconde extrémité est configurée pour s'enrouler en réponse au mouvement de l'élément d'avancement (22) de la position rétractée à la position d'avancement.

13. Dispositif d'accès vasculaire (10) selon la revendication 1, dans lequel la partie de support (62) comprend un couvercle (92) s'étendant sur la rainure (44), dans lequel un premier côté du couvercle (92) ou un second côté du couvercle (92) opposé au premier côté du couvercle (92) est couplé au boîtier (14) ou dans lequel un premier côté du couvercle (92) et le second côté du couvercle (92) opposé au premier côté sont couplés au boîtier (14), dans lequel le couvercle (92) comprend une fente vers le bas d'un milieu du couvercle ou l'élément d'avancement (22) comprend une lame (99) qui coupe à travers le couvercle (92) en réponse au mouvement de l'élément d'avancement (22) de la position rétractée à la position avancée.

14. Dispositif d'accès vasculaire (10) selon la revendication 1, dans lequel la partie de support (62) comprend une pluralité de pattes (102) s'étendant sur la rainure (44) et configurées pour pivoter ou se courber en réponse au mouvement de l'élément d'avancement (22) de la position rétractée à la position avancée et au contact par l'élément d'avancement (22).

15. Dispositif d'accès vasculaire (10) comprenant :
un boîtier (14), comprenant une extrémité proximale (16), une extrémité distale (18) et une fente (20), dans lequel une surface interne (42) du boîtier (14) comprend une rainure (44) disposée à l'intérieur du boîtier (14) entre l'extrémité proximale (16) du boîtier (14) et l'extrémité distale (18) du boîtier (14) ;
un élément d'avancement (22) s'étendant à travers la fente (20) et configuré pour se déplacer linéairement le long de la fente (20) entre une position rétractée et une position avancée ;
un instrument (12) disposé à l'intérieur de la rainure (44), l'instrument (12) comprenant une première extrémité (28) et une seconde extrémité (30), dans lequel, en réponse au mouvement de l'élément d'avancement (22) de la position rétractée à la position avancée, la seconde extrémité (30) de l'instrument (12) est avancée au-delà de l'extrémité distale (18) du boîtier (14) ; et
un tube (98) disposé à l'intérieur de la rainure (44), dans lequel l'instrument (12) s'étend à travers le tube (98), dans lequel :
le tube (98) comprend une fente (100) et en réponse au mouvement de l'élément d'avancement (22) de la position rétractée à la position avancée, l'élément d'avancement (22) se déplace distalement à travers la fente (100) ; ou
l'élément d'avancement (22) comprend un canal en forme d'arc (54) et en réponse au mouvement de l'élément d'avancement (22) de la position rétractée à la position avancée, le tube (98) coulisse proximalement dans le canal en forme d'arc (54).
